(19) Europäisches Patentamt
European Patent Office
Office européen des brevets

(11) Publication number: 0 122 761 B1

(12) EUROPEAN PATENT SPECIFICATION

(45) Date of publication of patent specification: 29.08.90

(21) Application number: 84302395.3

(22) Date of filing: 06.04.84

(51) Int. Cl.⁵: C 07 D 417/04, A 01 N 43/78, A 01 N 43/88, A 01 N 43/72

# (54) Herbicidal tetrahydrobenzothiazole derivatives.

(30) Priority: 08.04.83 JP 61925/83

(43) Date of publication of application: 24.10.84 Bulletin 84/43

(45) Publication of the grant of the patent: 29.08.90 Bulletin 90/35

(84) Designated Contracting States: DE FR GB IT

(56) References cited:
EP-A- 32 879
US-A-3 379 725
US-A-4 289 524

(73) Proprietor: KUREHA KAGAKU KOGYO KABUSHIKI KAISHA
9-11 Horidome-cho 1-chome Nihonbashi Chuo-ku
Tokyo 103 (JP)

(72) Inventor: Aoki, Katsumichi
16-10 Nishi-Arata Ueda-machi
Iwaki-shi Fukushima-ken (JP)
Inventor: Shida, Takafumi
1-6 Ochiai Nishiki-machi
Iwaki-shi Fukushima-ken (JP)
Inventor: Kumazawa, Satoru
16-1 Maehara Nishiki-machi
Iwaki-shi Fukushima-ken (JP)
Inventor: Shimizu, Susumu
111-2 Ayanouchi, Nishiki-machi
Iwaki-shi Fukushima-ken (JP)
Inventor: Kanda,Yoichi
45-2 Ohshima Nishiki-machi
Iwaki-shi Fukushima-ken (JP)
Inventor: Satake, Keigo
4-8-15 Nakaoka-machi
Iwaki-shi Fukushima-ken (JP)
Inventor: Yamazaki, Shiro
11-1 Kaneyama Joban-Nishigomachi
Iwaki-shi Fukushima-ken (JP)

**The file contains technical information submitted after the application was filed and not included in this specification**

EP 0 122 761 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: Shinkawa, Hiroyasu
14 Suka, Nishiki-machi
Iwaki-shi Fukushima-ken (JP)
Inventor: Chida, Tsuneaki
175-1 Kaminakada Nishiki-machi
Iwaki-shi Fukushima-ken (JP)
Inventor: Arabori, Hideo
16-1 Maehara Nishiki-machi
Iwaki-shi Fukushima-ken (JP)
Inventor: Watanabe, Takeo
78-31 Hananoi Nishiki-machi
Iwaki-shi Fukushima-ken (JP)

(74) Representative: Myerscough, Philip Boyd et al
J.A.Kemp & Co. 14, South Square Gray's Inn
London, WC1R 5EU (GB)

**Description**

The present invention relates to derivatives of tetrahydrobenzothiazole, their preparation, herbicidal compositions containing them and a method of controlling the growth of weeds using them.

The present inventors have sought to find a compound showing excellent activity in selectively controlling weeds such as *Echinochloa crus-galli, Poa annua, Cardamine flexuosa, Portulaca orelacea*, without being phytotoxic to crop plants such as rice, wheat, soy-bean and maize. As a result, they have found that derivatives of tetrahydrobenzothiazole represented by the formula (I) below show excellent herbicidal activity for practically controlling the weeds.

US—A—4,289,524 discloses compounds having herbicidal activity of formula:

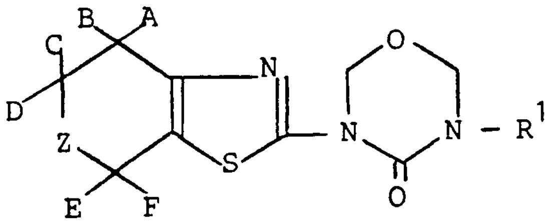

wherein E is hydrogen or a $C_1$—$C_{10}$ alkyl group and F is a $C_1$—$C_{10}$ alkyl, $C_5$—$C_{10}$ alkenyl, $C_4$—$C_{10}$ alkynyl, cycloalkyl, alkoxy, alkylthio, amino or cyano group or hydrogen.

EP—A—32,829 discloses compounds having herbicidal activity of formula:

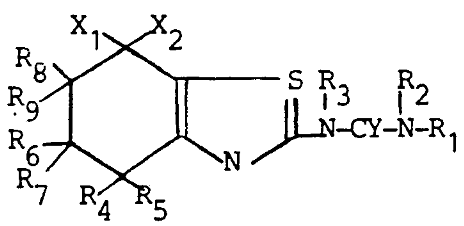

wherein $X_1$ and $X_2$ may together form a =O group.

US—A—3,379,725 discloses compounds having herbicidal activity of formula:

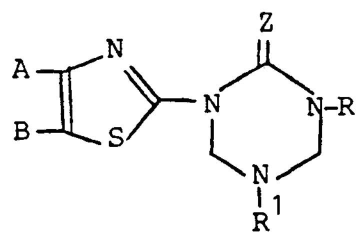

wherein Z is oxygen or sulphur, R is a hydrocarbon radical containing 1 to 4 carbon atoms, $R^1$ is a hydrocarbon radical containing 1 to 6 carbon atoms and A and B are hydrogen atoms or hydrocarbon radicals containing 1 to 4 carbon atoms or A and B together represent a hydrocarbon bridge member containing 3 to 5 carbon atoms.

The compounds represented by the formula (I) are novel compounds, and of course, their physiological properties have never been known. According to herbicidal tests consisting essentially of foliar application and soil treatment, each of the derivatives of tetrahydrobenzothiazole according to the present invention (hereinafter referred to as "the present compounds") shows an excellent herbicidal activity on broad-leaved weeds, for instance, *Stellaria media, Cardamine flexuosa* and *Portulaca oleracea*, Cyperaceous weeds, for instance, *Cyperus iria* and Gramineous weeds, for instance, those belonging to the genus *Echinochloa* and *Poa annua* and particularly shows strong herbicidal activity when applied on leaves and stems of these weeds. The application is carried out on arable lands such as paddy fields, upland fields and orchards and also on non-arable lands.

In a first aspect of the present invention, there is provided a derivative of tetrahydrobenzothiazole represented by the formula (I):

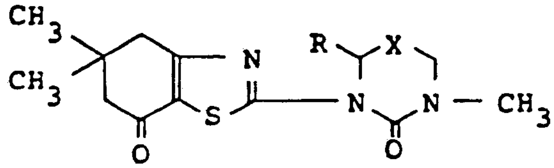

(I)

wherein R represents a hydrogen atom, a hydroxy group or a $C_2$—$C_6$ acyloxy group, such as acetoxy or propionyloxy group, and X represents a methylene group, a ($C_1$ to $C_4$-alkyl)N< group, a (benzyl)N< group or —O—.

In a second aspect of the present invention, there is provided a herbicidal composition comprising as active ingredient a compound of formula (I), together with a carrier or diluent therefor.

In the attached Drawing, Figs. 1 to 11 show the infrared absorption spectra of Compounds Nos. 1 to 11 according to the present invention, respectively.

The compounds of formula (I) are synthesized from the compounds of formula (II) or (III).

Thus the present invention also provides a process for the preparation of a compound of formula (I), which process comprises:—

(i) reacting formaldehyde and a $C_1$—$C_4$ alkylamine or benzylamine with a compound of formula (II):

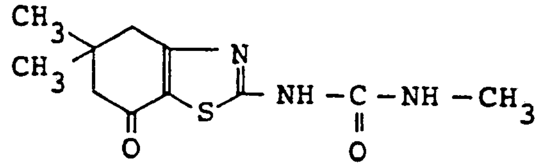
(II)

to obtain a compound of formula (I) in which R represents a hydrogen atom and X represents a ($C_1$—$C_4$alkyl)N< or (benzyl)N< group; or

(ii) reacting a compound of formula (II) with trioxane, preferably in the presence of an acid catalyst, to obtain a compound of formula (I) in which R represents a hydrogen atom and X represents —O—.

In process (i), 2 mols of formaldehyde and 1 mol of a $C_1$ to $C_4$-alkylamine, or 1 mol of benzylamine are reacted per 1 mol of the compound of formula (II).

The present invention further provides a process for the preparation of a compound of formula (I) in which R represents a hydroxyl group or a $C_2$—$C_6$ acyloxy group and X represents a methylene group, which process comprises treating a compound of formula (III).

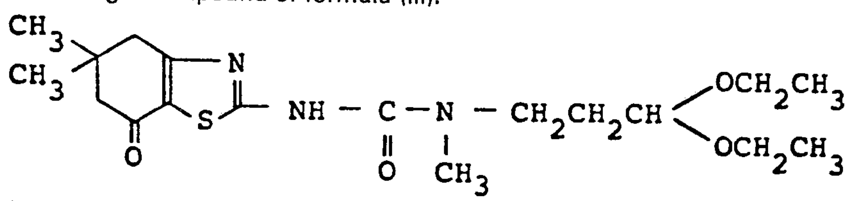
(III)

with an aqueous dilute solution of a mineral acid to obtain a compound of formula (I) in which R represents a hydroxyl group and X represents a methylene group, ie a compound of formula (VI):

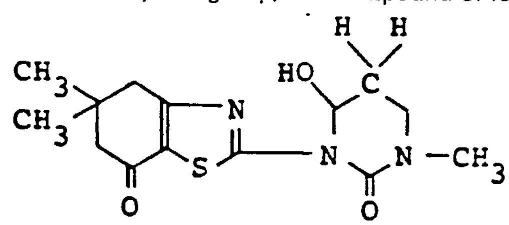
(VI)

and, if desired, converting the hydroxyl group into a $C_2$—$C_6$ acyloxy group to obtain a compound of formula (VII):

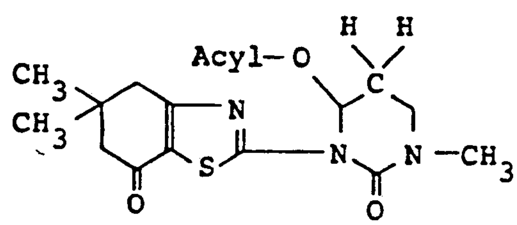
(VII)

The compound represented by the formula (II) is easily made by reacting the compound represented by the formula (IV) with methyl isocyanate as follows:

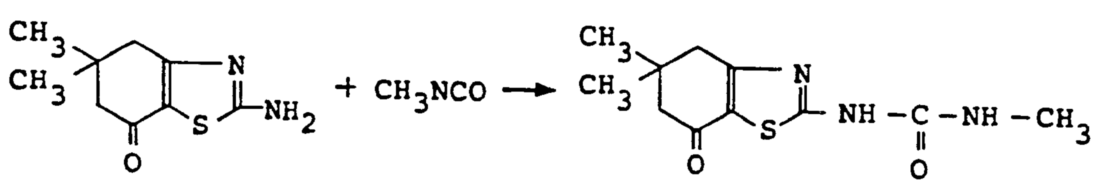

(IV) (II)

The compound represented by the formula (III) is easily synthesized by reacting (3,3-diethoxypropyl)-methylamine with a carbamate represented by the formula (V) which is obtained by reacting phenyl chloroformate with the compound represented by the formula (IV) as follows.

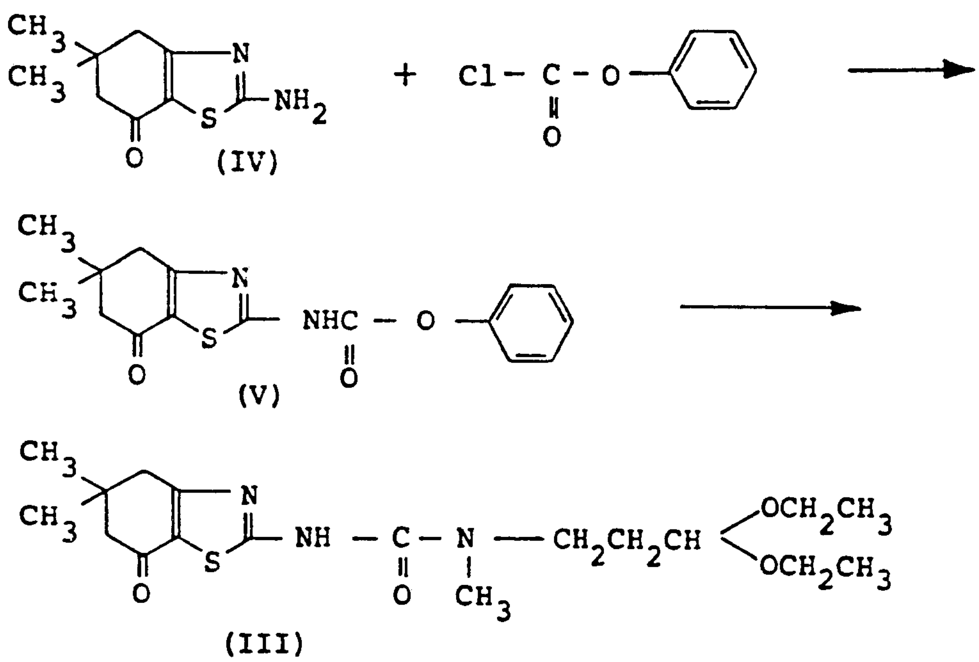

(IV)

(V)

(III)

Examples of the present compounds and the physicochemical properties thereof are shown in Table 1.

Table 1

| Compound number | 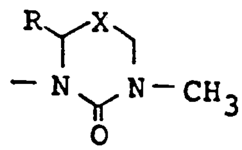 | Melting point (°C) | Yield (%) | Infrared absorption spectrum |
|---|---|---|---|---|
| 1 | 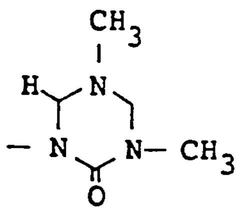 | 182 - 184 | 80 | Fig. 1 |
| 2 | 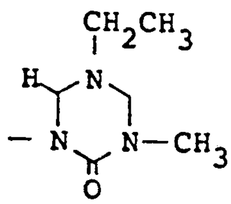 | 153 - 155 | 84 | Fig. 2 |
| 3 | 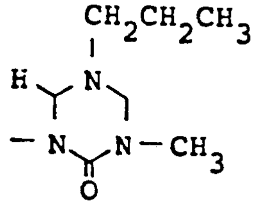 | 142 - 143 | 71 | Fig. 3 |
| 4 | 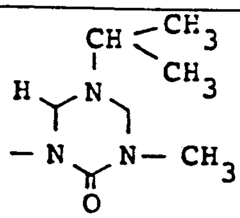 | 125 - 127 | 86 | Fig. 4 |
| 5 | 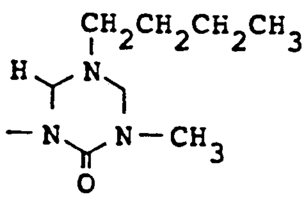 | 129 - 131 | 87 | Fig. 5 |

Continued

Table 1 (continued)

| | | | | |
|---|---|---|---|---|
| 6 | 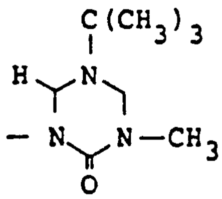 | 157 - 159 | 88 | Fig. 6 |
| 7 | 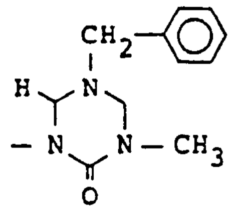 | 166 - 167 | 71 | Fig. 7 |
| 8 | 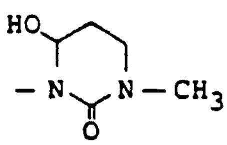 | 163 - 165 | 67 | Fig. 8 |
| 9 | 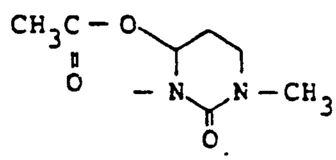 | 170 - 172 | 46 | Fig. 9 |
| 10 | 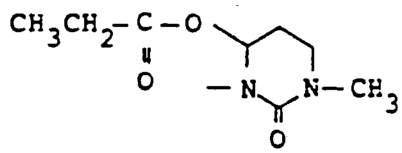 | 123 - 125 | 36 | Fig. 10 |
| 11 | 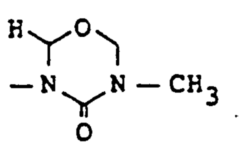 | 146 - 147 | 34 | Fig. 11 |

The present invention finally provides a method of controlling the growth of weeds at a locus, which method comprises applying to the locus a herbicidally effective amount of a compound of formula (I).

The present invention will be more precisely explained in the following examples.

The NMR specta of the compounds were taken using TMS as the internal standard. The following symbols are used:

s means a singlet; d means a doublet; t means a triplet;
6-plet means sextet and m means multiplet.

Example 1

Synthesis of tetrahydro-1-(4,5,6,7-tetrahydro-5,5-dimethyl-7-oxo-2-benzothiazolyl)-3,5-dimethyl-1,3,5-triazin-2(1H)-one (Compound No. 1)

Into a solution of 3.0 g (0.012 mol) of N-(4,5,6,7-tetrahydro-5,5-dimethyl-7-oxo-2-benzothiazolyl)-N'-methylurea in 16 ml of dimethylformamide, 3.1 ml of aqueous 35% solution of formaldehyde (0.036 mol) was added, and the mixture was stirred for 30 min at room temperature. Then, 1.5 ml of aqueous 40%

solution of methylamine (0.019 mol) was added to the mixture, and the whole mixture was stirred for 16 hours at room temperature and for a further 3 hours at 90°C.

After distilling the solvent off from the reaction mixture, the residual solid matter was recrystallized from benzene to obtain 2.9 g of pale yellow crystals melting at 182 to 184°C in a yield of 80%. The product showed the following IR spectrum and NMR spectrum.

IR(KBr, $cm^{-1}$): $\nu_{CO}$ 1670 and 1640.

NMR($CDCl_3$)δ(ppm): 1.15 [6H, s, 5'-$(CH_3)_2$]; 2.43 and 2.79 (each 2H, each s, 4'-H and 6'-H); 2.70 (3H, s, 5-$CH_3$); 3.06 (3H, s, 3-$CH_3$); 4.37 (2H, s, 4-$CH_2$) and 5.20 (2H, s, 6-$H_2$).

Example 2

Synthesis of tetrahydro-1-(4,5,6,7-tetrahydro-5,5-dimethyl-7-oxo-2-benzothiazolyl)-6-hydroxy-3-methyl-2(1H)-pyrimidinone (Compound No. 8)

Into 50 ml of aqueous 5% solution of sulfuric acid, 3.8 g (0.01 mol) of N-(3,3-diethoxypropyl)-N'-(4,5,6,7-tetrahydro-5,5-dimethyl-7-oxo-2-benzothiazolyl)-N-methylurea was added, and the mixture was stirred for 1 hour at 70 to 80°C. After cooling the reaction mixture, the precipitated crystals were collected by filtration, and after washing the crystals with water, the crystals were recrystallized from aqueous 50% ethanolic solution to obtain 2.0 g of white crystals melting at 163 to 165°C in a yield of 67%. The product showed the following IR spectrum and NMR spectrum.

IR(KBr, $cm^{-1}$): $\nu_{OH}$ 3200, $\nu_{CO}$ 1640.

NMR($CDCl_3$)δ(ppm): 1.10 [6H, s, 5'-$(CH_3)_2$]; 2.04—2.43 (2H, m, 5-$H_2$); 2.41 and 2.73 (each 2H, each s, 4'-$H_2$ and 6'-$H_2$); 3.10 (3H, s, 1-$CH_3$); 3.20—4.11 (2H, m, 6-$H_2$); 5.12 (1H, bs, OH) and 6.18 (1H, t, J=3Hz, 4-H).

Example 3

Synthesis of 6-acetoxytetrahydro-1-(4,5,6,7-tetrahydro-5,5-dimethyl-7-oxo-2-benzothiazolyl)-3-methyl 2(1H)-pyrimidinone (Compound No. 9)

Into 20 ml of pyridine, 1.0 g (0.0032 mol) of tetrahydro-1-(4,5,6,7-tetrahydro-5,5-dimethyl-7-oxo-2-benzothiazolyl)-6-hydroxy-3-methyl-2(1H)-pyrimidinone (Compound No. 8 synthesized in Example 2) was dissolved, and 0.8 g (0.01 mol) of acetyl chloride was added dropwise to the solution at 0 to 5°C. After stirring the reaction mixture for 3 hours at this temperature, the reaction mixture was poured into iced water, and extracted with chloroform. After washing the organic layer with a dilute hydrochloric acid and then with water, and drying the washed organic layer, the solvent was removed to obtain an oily substance, which was purified by silica gel chromatography to obtain 0.5 g of white crystals melting at 170 to 172°C in a yield of 46%. The product showed the following IR spectrum and NMR spectrum.

IR(KBr, $cm^{-1}$): $\nu_{CO}$ 1730, 1670 and 1650.

NMR($CDCl_3$)δ(ppm): 1.11 [6H, s, 5'-$(CH_3)_2$]; 1.98—2.52 (2H, m, 5-$H_2$); 2.09 (3H, s, $OCOCH_3$); 2.42 and 2.77 (each 2H, each s, 4'-$H_2$ and 6'-$H_2$); 3.12 (3H, s, 1-$CH_3$); 3.10—4.02 (2H, m, 6-$H_2$) and 7.49 (1H, t, J=3Hz, 4—H).

Example 4

Synthesis of tetrahydro-3-(4,5,6,7-tetrahydro-5,5-dimethyl-7-oxo-2-benzothiazolyl)-5-methyl-4H-1,3,5-oxadiazin-4-one (Compound No. 11)

Into 20 ml of concentrated sulfuric acid, 2.0 g (0.008 mol) of N-(4,5,6,7-tetrahydro-5,5-dimethyl-7-oxo-2-benzothiazolyl)-N'-methylurea and 1.4 g (0.016 mol) of trioxane were added, and the mixture was stirred for 10 hours at room temperature. After pouring the reaction mixture into iced water, the mixture was extracted with chloroform, and the organic layer was washed with water and dried. The solvent was removed to obtain a solid matter, which was purified by silica gel-chromatography to obtain 0.8 g of white crystals melting at 146 to 147°C in a yield of 34%. The product showed the following IR spectrum and NMR spectrum.

IR(KBr, $cm^{-1}$): $\nu_{CO}$ 1665 and 1635.

NMR($CDCl_3$)δ(ppm): 1.10 [6H, s, 5'-$(CH_3)_2$]; 2.40 and 2.73 (each 2H, each s, 4'-$H_2$ and 6'-$H_2$); 3.04 (3H, s, 5-$CH_3$); 5.03 (2H, s, 6-$H_2$) and 5.70 (2H, s, 2-$H_2$).

Preparation Example 1

Preparation and Application of a Wettable Powder

By mixing 50 parts by weight of the present compound (Compound No. 2), 5 parts by weight of a salt of ligninsulfonic acid, 3 parts by wight of a salt of alkylsulfonyl acid and 42 parts by weight of diatomaceous earth, and pulverizing the mixture, a wettable powder was prepared.

The thus prepared wettable powder is applied after diluting thereof with water to a suitable concentration of Compound No. 2 as the active ingredient.

Preparation Example 2

Preparation and Application of an Emulsifiable Concentrate

By uniformly mixing 25 parts by weight of the present compound (Compound No. 8 synthesized in Example 2), 65 parts by weight of xylene and 10 parts by weight of polyoxyethylenealkyl aryl ether, an emulsifiable concentrate was prepared.

The thus prepared emulsifiable concentrate is applied after diluting thereof with water to a suitable concentration of Compound No. 8 as the active ingredient.

Preparation Example 3

Preparation and Application of a Granular composition

After uniformly mixing 8 parts by weight of the present compound (Compound No. 11 synthesized in Example 4), 40 parts by weight of bentonite, 45 parts by weight of clay and 7 parts by weight of a salt of ligninsulfonic acid, the mixture was kneaded with water and processed into granules by an extruding granulator. The granules were dried and sifted to be a product of granular composition which is directly applied.

The effectiveness of the present compounds is shown below.

Herbicidal Test Example

To the folliage of each of the following plants grown from their seeds under a management in a plastic planter of 180 × 580 × 150 mm in size of width, length and height filled with a soil collected from an actual crop field, each of the wettable powders prepared as in Preparation Example 1 and diluted to 0.1% by weight of the active ingredient therein with water and the thus prepared aqueous suspension was sprayed by a small pressured-sprayer at a rate of 10 litres per acre (100 $m^2$). After spraying the plastic planters were placed in a green house.

After 20 days of the application, the state of the plants was observed to assess the damage due to the application of each of the wettable powders, thereby finding out the herbicidally activity thereof according to the following criteria.

Criteria of herbicidal activity

| Index | Damage (phytotoxicity) |
|---|---|
| 0 | none |
| 1 | very slight |
| 2 | slight |
| 3 | moderate |
| 4 | severe |
| 5 | very severe (plant withered) |

Name of plants

| | |
|---|---|
| 1. *Echinochloa crus-galli* | 2. *Digitaria ciliaris* |
| 3. *Poa annua* | 4. *Cyperus iria* |
| 5. *Chenopodium album* | 6. *Stellaria media* |
| 7. *Cardamine flexuosa* | 8. *Portulaca oleracea* |
| 9. *Glycine max* (soybean) | 10. *Zea mays* (maize) |
| 11. *Triticum aestivum* (wheat) | |

The herbicidal activities of the present compounds thus assessed are shown in Table 2. The growth state of the plants when the present test was carried out was the 2 to 4 leaf-stage.

Table 2: Herbicidal Activity

| Plant \ Present compound | 1 | 2 | 3 | 4 | 5 | 6 | 7 | 8 | 9 | 10 | 11 | not applied |
|---|---|---|---|---|---|---|---|---|---|---|---|---|
| *Echinochloa curs-galli* | 3 | 5 | 5 | 3 | 2 | 3 | 2 | 3 | 3 | 3 | 5 | 0 |
| *Digitaria ciliaris* | 4 | 5 | 5 | 3 | 1 | 4 | 2 | 3 | 1 | 2 | 5 | 0 |
| *Poa annua* | 3 | 5 | 5 | 3 | 2 | 5 | 2 | 4 | 3 | 5 | 4 | 0 |
| *Cyperus iria* | 3 | 3 | 3 | 3 | 2 | 1 | 2 | 2 | 2 | 2 | 4 | 0 |
| *Chenopodium album* | 4 | 5 | 5 | 5 | 4 | 5 | 1 | 5 | 5 | 5 | 5 | 0 |
| *Stellaria media* | 5 | 5 | 5 | 5 | 5 | 5 | 3 | 5 | 4 | 4 | 5 | 0 |
| *Cardamine flexuosa* | 5 | 5 | 5 | 5 | 4 | 5 | 3 | 5 | 5 | 5 | 5 | 0 |
| *Portulaca oleracea* | 4 | 5 | 5 | 5 | 3 | 5 | 2 | 5 | 5 | 2 | 5 | 0 |
| *Glycine max* (soybean) | 0 | 0 | 2 | 0 | 0 | 0 | 0 | 1 | 0 | 0 | 2 | 0 |
| *Zea mays* (maize) | 0 | 0 | 2 | 0 | 0 | 0 | 0 | 0 | 0 | 0 | 0 | 0 |
| *Triticum aestivum* (wheat) | 0 | 2 | 0 | 1 | 0 | 1 | 0 | 1 | 1 | 1 | 2 | 0 |

**Claims**

1. A derivative of tetrahydrobenzothiazole, represented by the formula (I):

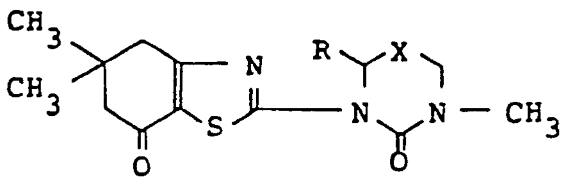

(I)

wherein R represents a hydrogen atom, a hydroxyl group or a $C_2$—$C_6$ acyloxy group and X represents a methylene group, a ($C_1$ to $C_4$-alkyl)N< group, a (benzyl)N< group or —O—.

2. A compound according to claim 1 in which R represents an acetoxy or propionyloxy group.

3. A compound according to claim 1 which is tetrahydro-1-(4,5,6,7-tetrahydro-5,5-dimethyl-7-oxo-2-benzothiazolyl)-3,5-dimethyl-1,3,5-triazin-2(1H)-one, represented by the formula:

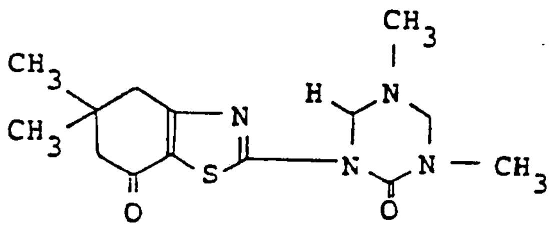

4. A compound according to claim 1 which is tetrahydro-1-(4,5,6,7-tetrahydro-5,5-dimethyl-7-oxo-2-benzothiazolyl)-3-methyl-5-n-propyl-1,3,5-triazin-2(1H)-one, represented by the formula:

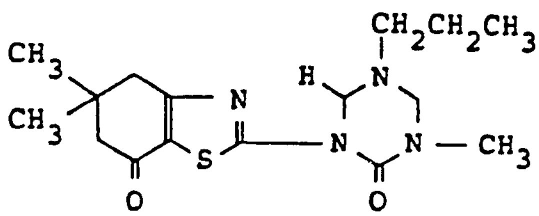

5. A compound according to claim 1 which is tetrahydro-1-(4,5,6,7-tetrahydro-5,5-dimethyl-7-oxo-2-benzothiazolyl)-3-methyl-5-isopropyl-1,3,5-triazin-2(1H)-one, represented by the formula:

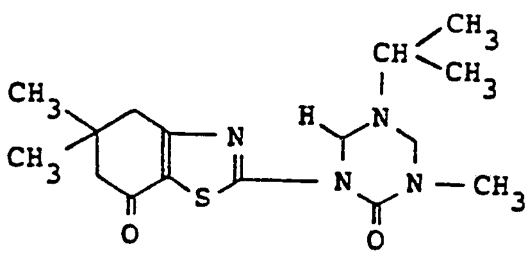

6. A compound according to claim 1 which is tetrahydro-3-(4,5,6,7-tetrahydro-5,5-dimethyl-7-oxo-2-benzothiazolyl)-5-methyl-4(H)-1,3,5-oxadiazin-4-one, represented by the formula:

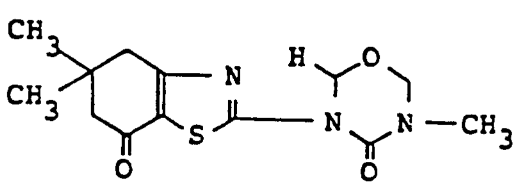

7. A process for the preparation of a compound of formula (I) as defined in claim 1, which process comprises:

(i) reacting formaldehyde and a $C_1$—$C_4$ alkylamine or benzylamine with a compound of formula (II):

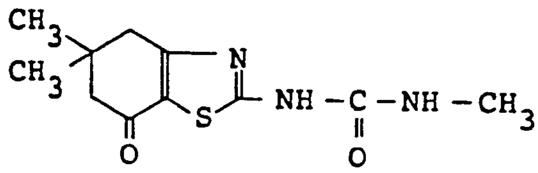

(II)

to obtain a compound of formula (I) in which R represents a hydrogen atom and X represents a ($C_1$—$C_4$ alkyl)N< or (benzyl)N< group; or

(ii) reacting a compound of formula (II) with trioxane to obtain a compound of formula (I) in which R represents a hydrogen atom and X represents —O—.

8. A process for the preparation of a compound of formula (I) as defined in claim 1 in which R represents a hydroxyl group or a $C_2$—$C_6$ acyloxy group and X represents a methylene group, which process comprises treating a compound of formula (III):

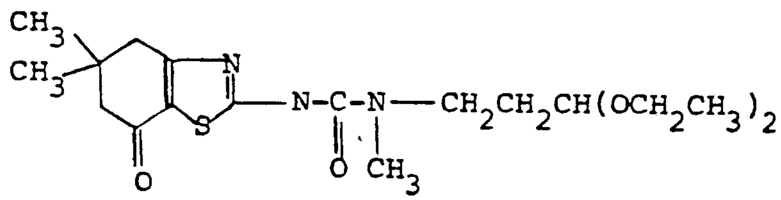

(III)

with an aqueous dilute solution of a mineral acid to obtain a compound of formula (I) in which R represents a hydroxyl group and X represents a methylene group and, if desired, converting the hydroxyl group into a $C_2$—$C_6$ acyloxy group.

9. A herbicidal composition comprising as active ingredient a compound of formula (I) as defined in claim 1, together with a carrier or diluent therefor.

10. A method of controlling the growth of weeds at a locus, which method comprises applying to the locus a herbicidally effective amount of a compound of formula (I) as defined in claim 1.

**Patentansprüche**

1. Tetrahydrobenzothiazol-Derivat, dargestellt durch die Formel (I)

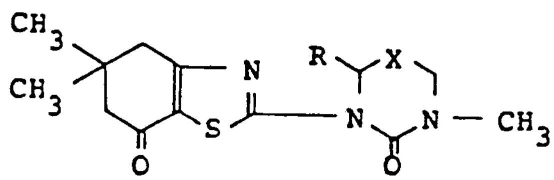

(I)

worin R ein Wasserstoffatom, eine Hydroxylgruppe oder eine $C_2$—$C_6$-Acyloxygruppe und X eine Methylengruppe, ($C_1$ bis $C_4$-Alkyl)N< Gruppe, eine (Benzyl)N< Gruppe oder —O— bedeuten.

2. Verbindung nach Anspruch 1, wobei R eine Acetoxy- oder Propionyloxygruppe bedeutet.

3. Verbindung nach Anspruch 1, nämlich Tetrahydro-1-(4,5,6,7-tetrahydro-5,5-dimethyl-6-oxo-2-benzothiazolyl)-3,5-dimethyl-1,3,5-triazin-2(1H)-on, dargestellt durch die Formel

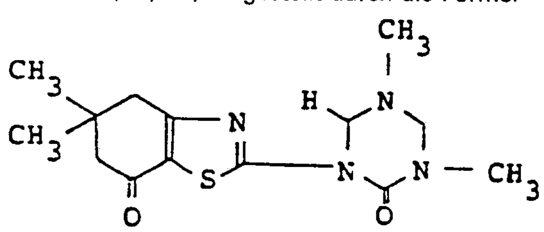

4. Verbindung nach Anspruch 1, nämlich Tetrahydro-1-(4,5,6,7-tetrahydro-5,5-dimethyl-7-oxo-2-benzothiazolyl)-3-methyl-5-n-propyl-1,3,5-triazin-2(1H)-on, dargestellt durch die Formel

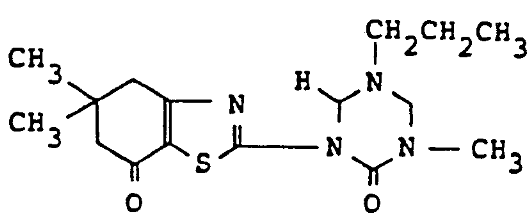

5. Verbindung nach Anspruch 1, nämlich Tetrahydro-1-(4,5,6,7-tetrahydro-5,5-dimethyl-7-oxo-2-benzothiazolyl)-3-methyl-5-isopropyl-1,3,5-triazin-2(1H)-on, dargestellt durch die Formel

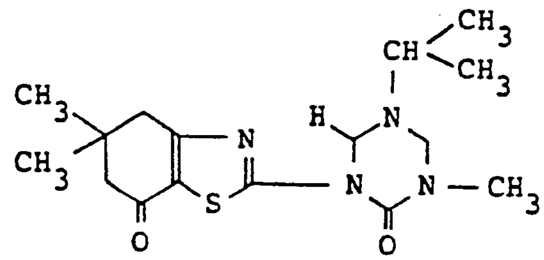

6. Verbindung nach Anspruch 1, nämlich Tetrahydro-3-(4,5,6,7-tetrahydro-5,5-dimethyl-7-oxo-2-benzothiazolyl)-5-methyl-4(H)-1,3,5-oxadiazin-4-on, dargestellt durch die Formel

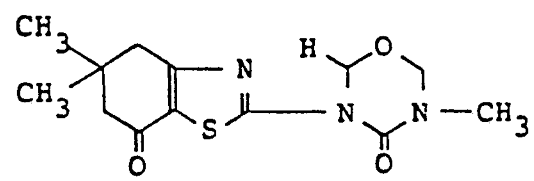

7. Verfahren zur Herstellung einer Verbindung der Formel (I), wie in Anspruch 1 definiert, umfassend:

(i) Umsetzen von Formaldehyd und einem $C_1$—$C_4$-Alkylamin oder Benzylamin mit einer Verbindung der Formel (II):

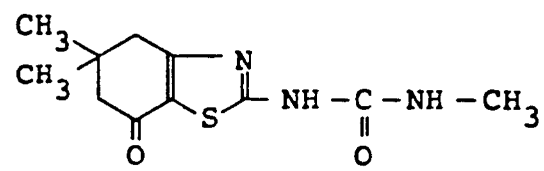

(II)

um eine Verbindung der Formel (I) zu erhalten, in der R ein Wasserstoffatom und X eine ($C_1$—$C_4$-Alkyl)N< oder (Benzyl)N< Gruppe darstellen; oder

(ii) Umsetzen einer Verbindung der Formel (II) mit Trioxan, um eine Verbindung der Formel (I) zu erhalten, in der R ein Wasserstoffatom und X —O— darstellen.

8. Verfahren zur Herstellung einer Verbindung der Formel (I), wie in Anspruch 1 definiert, wobei R eine Hydroxygruppe oder eine $C_2$—$C_6$-Acyloxygruppe und X eine Methylengruppe bedeuten, umfassend das Behandeln einer Verbindung der Formel (III)

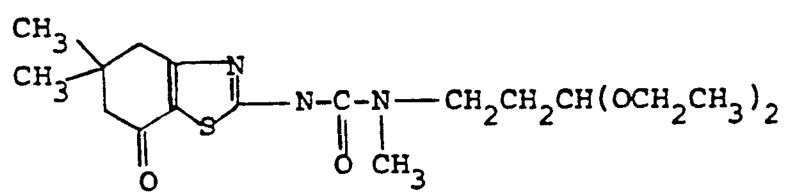

(III)

mit einer wäßrigen verdünnten Lösung einer Mineralsäure, um eine Verbindung der Formel (I) zu erhalten, in der R eine Hydroxylgruppe und X eine Methylengruppe darstellen und, falls erwünscht, Überführen der Hydroxylgruppe in eine $C_2$—$C_6$-Acyloxygruppe.

9. Herbizid wirksame Zusammensetzung, umfassend als wirksamen Bestandteil eine Verbindung der Formel (I), wie in Anspruch 1 definiert, zusammen mit einem Träger oder Verdünnungsmittel hierfür.

10. Verfahren zur Regulierung des Wachstums von Unkraut an einer Stelle, wobei das Verfahren die Anwendung an der Stelle einer herbizid wirksamen Menge einer Verbindung der Formel (I), wie in Anspruch 1 definiert, umfaßt.

**Revendications**

1. Dérivé de tétrahydrobenzothiazole représenté par la formule (I):

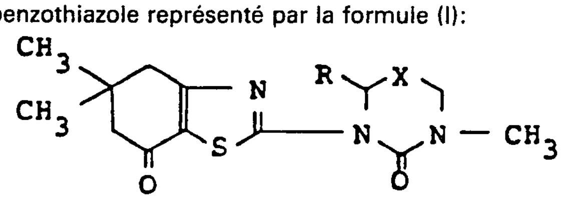

(I)

dans laquelle R représente un atome d'hydrogène, le groupe hydroxy ou un groupe acyloxy en $C_2$—$C_6$, et X représenté le groupe méthylène, un groupe alkyl($C_1$—$C_4$)N<, un groupe (benzyl)N< ou —O—.

2. Composé selon la revendication 1, dans lequel R représente le groupe acétoxy ou propionyloxy.

3. Composé selon la revendication 1, lequel est la tétrahydro-1-(4,5,6,7-tétrahydro-5,5-diméthyl-7-oxo-2-benzothiazolyl)-3,5-diméthyl-1,3,5-triazine-2(1H)-one, représentée par la formule:

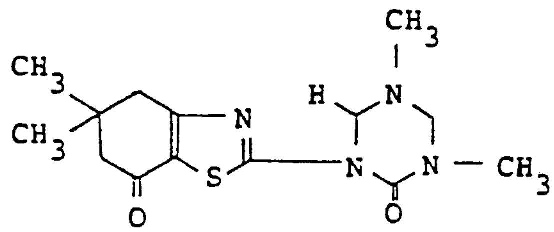

4. Composé selon la revendication 1, lequel est la tétrahydro-1-(4,5,6,7-tétrahydro-5,5-diméthyl-7-oxo-2-benzothiazolyl)-3-méthyl-5-n-propyl-1,3,5-triazine-2(1H)-one, représentée par la formule:

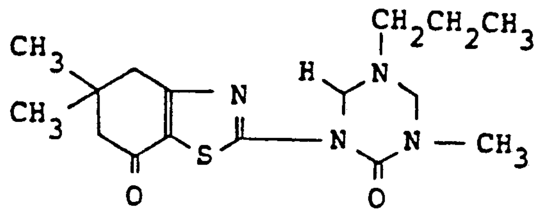

5. Composé selon la revendication 1, lequel est la tétrahydro-1-(4,5,6,7-tétrahydro-5,5-diméthyl-7-oxo-2-benzothiazolyl)-3-méthyl-5-isopropyl-1,3,5-triazine-2(1H)-one, représentée par la formule:

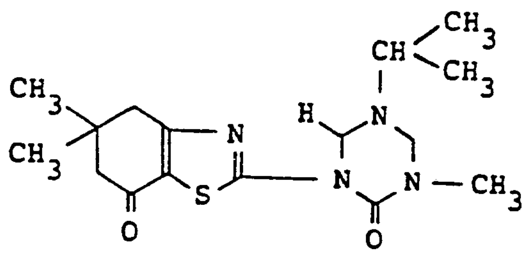

6. Composé selon la revendication 1, lequel est la tétrahydro-3-(4,5,6,7-tétrahydro-5,5-diméthyl-7-oxo-2-benzothiazolyl)-5-méthyl-4(H)-1,3,5-oxadiazine-4-one, représentée par la formule:

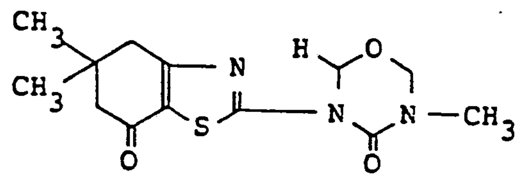

7. Procédé pour la préparation d'un composé de formule (I) tel que défini dans la revendication 1, lequel procédé comprend:

(I) la mise en réaction du formaldéhyde et d'une alkylamine en $C_1$—$C_4$ ou de la benzylamine avec un composé de formule (II):

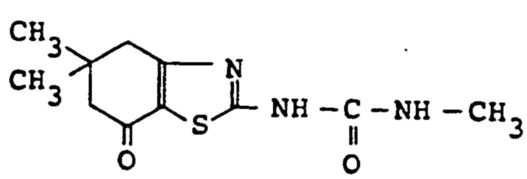

(II)

pour l'obtention d'un composé de formule (I) dans lequel R représente un atome d'hydrogène et X représente un groupe alkyl($C_1$—$C_4$)N< ou (benzyl)N<; ou

(II) la mise en réaction d'un composé de formule (II) avec du trioxanne, pour l'obtention d'un composé de formule (I) dans lequel R représente un atome d'hydrogène et X représente —O—.

8. Procédé pour la préparation d'un composé de formule (I) tel que défini dans la revendication 1, dans lequel R représente le groupe hydroxy ou un groupe acyloxy en $C_2$—$C_6$, et X représente le groupe méthylène, lequel procédé comprend le traitement d'un composé de formule (III):

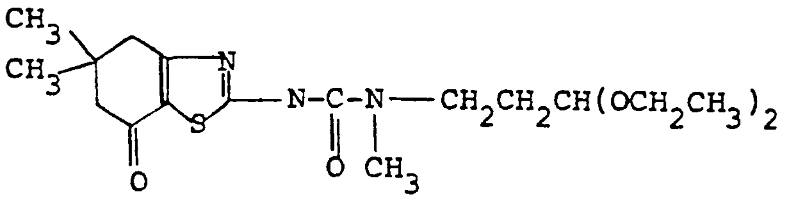

(III)

par une solution aqueuse diluée d'un acide minéral, pour l'obtention d'un composé de formule (I) dans lequel R représente le groupe hydroxy et X représente le groupe méthylène, et, si on le désire, la conversion du groupe hydroxy en un groupe acyloxy en $C_2$—$C_6$.

9. Composition herbicide comprenant en tant que composant actif un composé de formule (I) tel que défini dans la revendication 1, conjointement avec un support ou diluant pour celui-ci.

10. Procédé de lutte contre le croissance de mauvaises herbes en un lieu, lequel procédé comprend l'application sur le lieu d'un quantité efficace en tant qu'herbicide d'un composé de formule (I) tel que défini dans la revendication 1.

Fig. 1

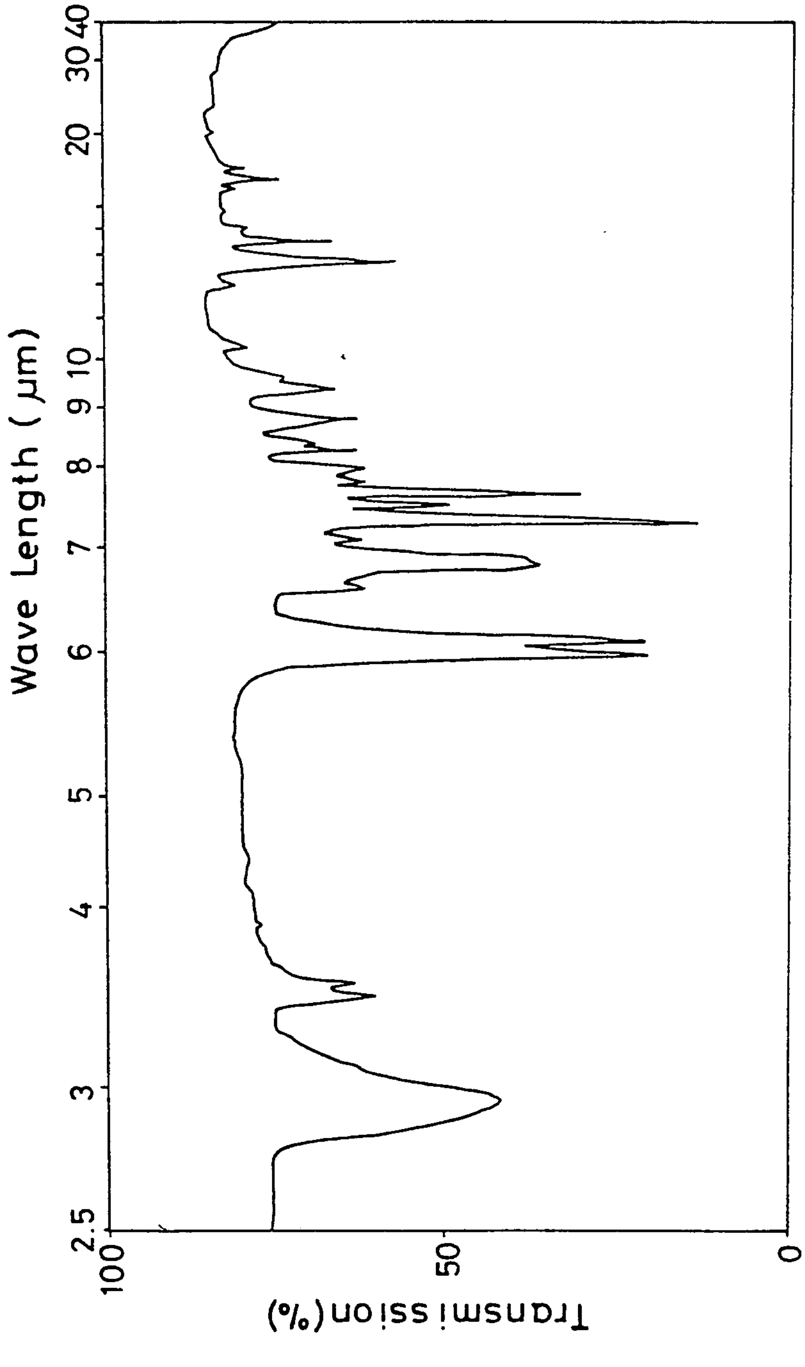

Fig. 2

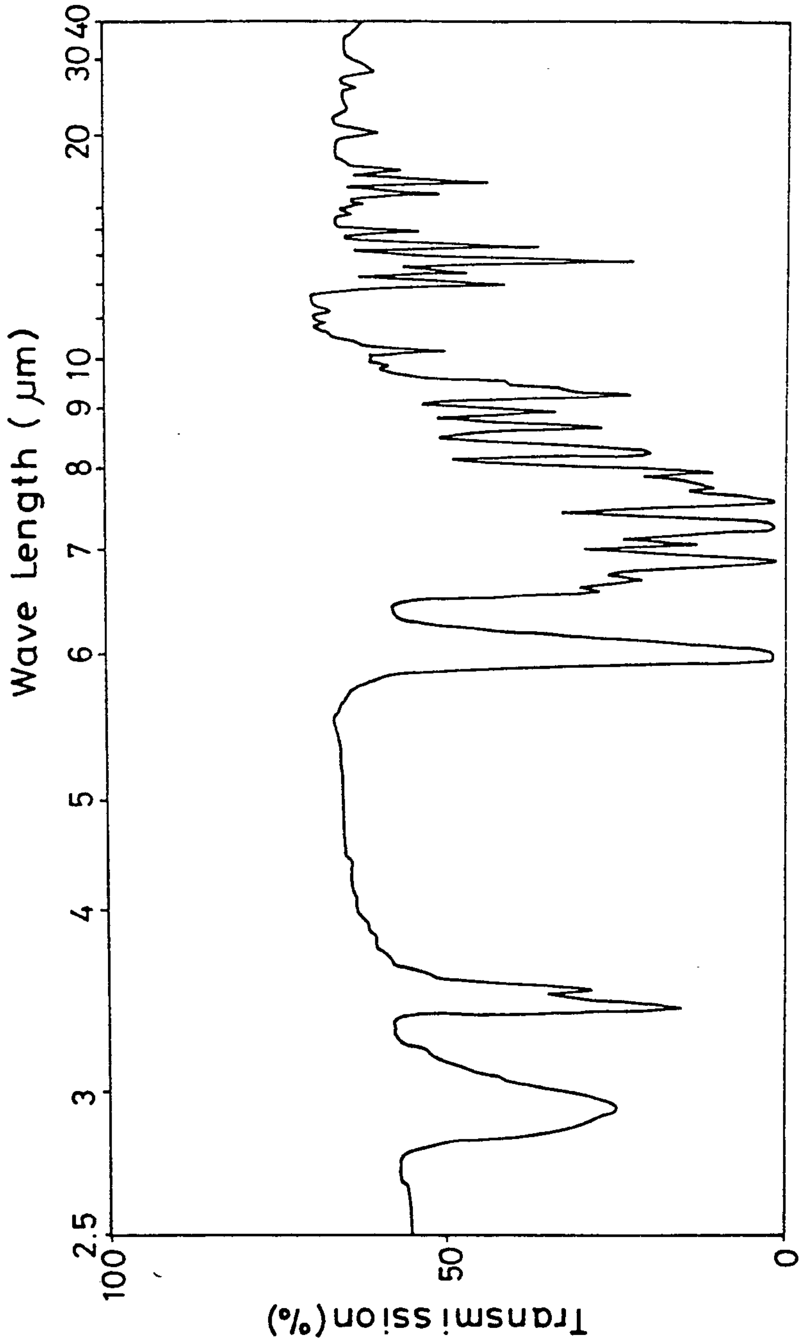

Fig. 3

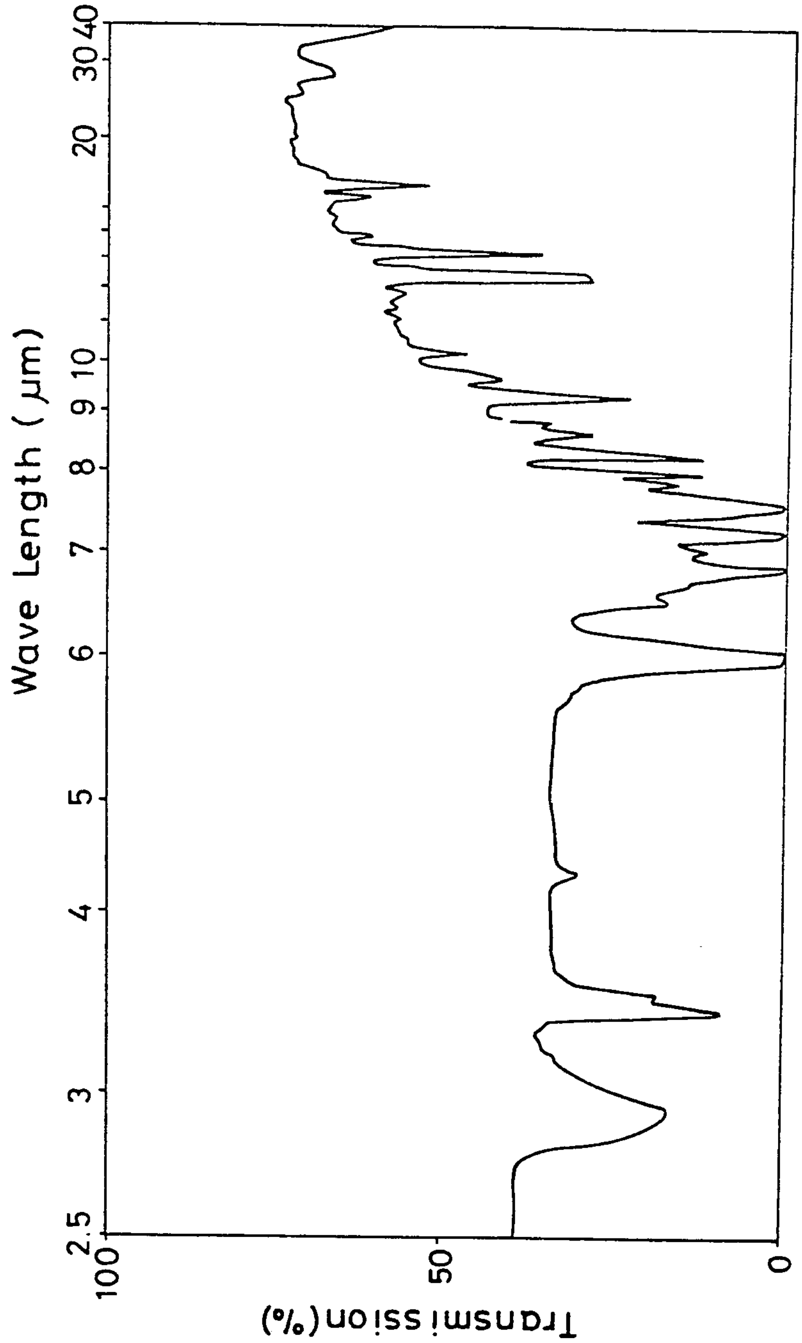

Fig. 4

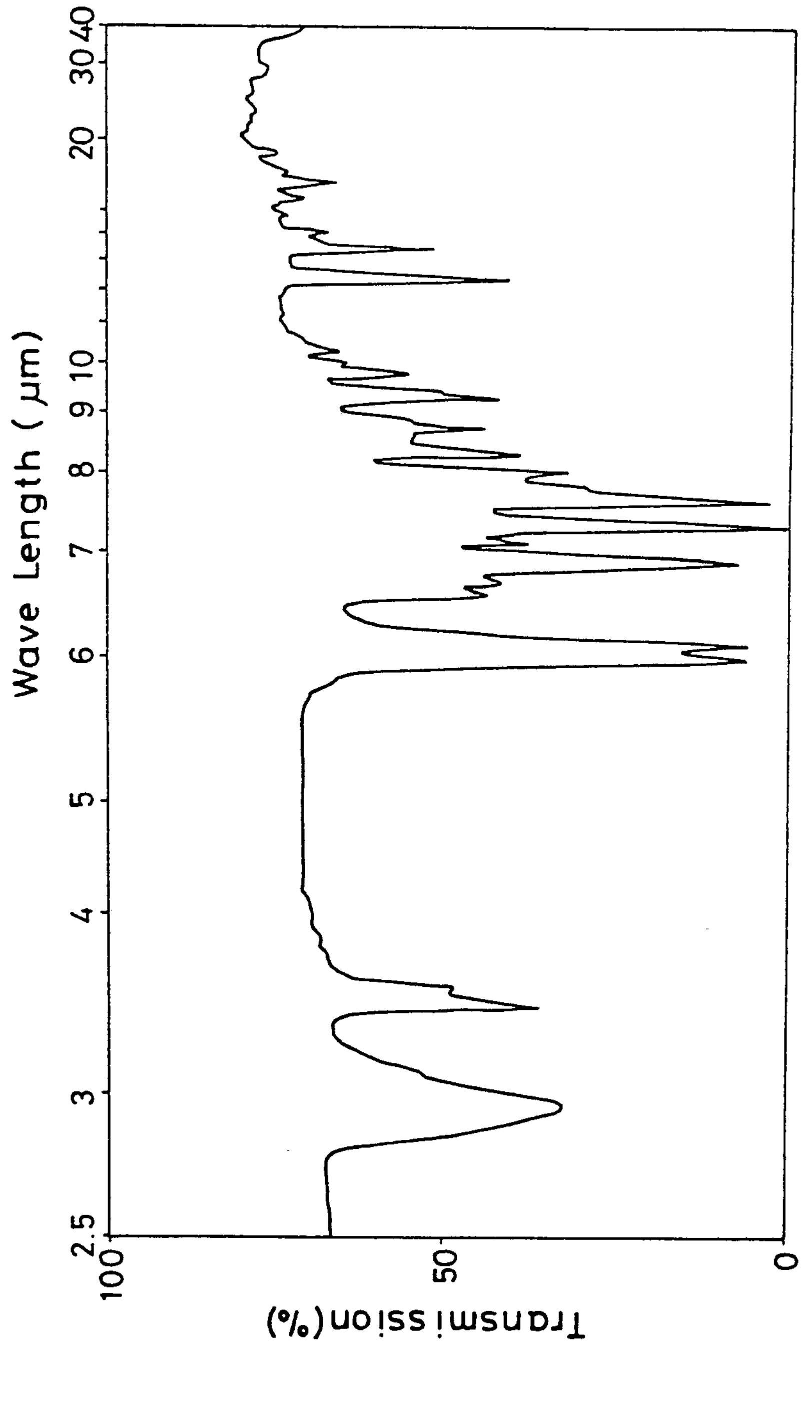

Fig. 5

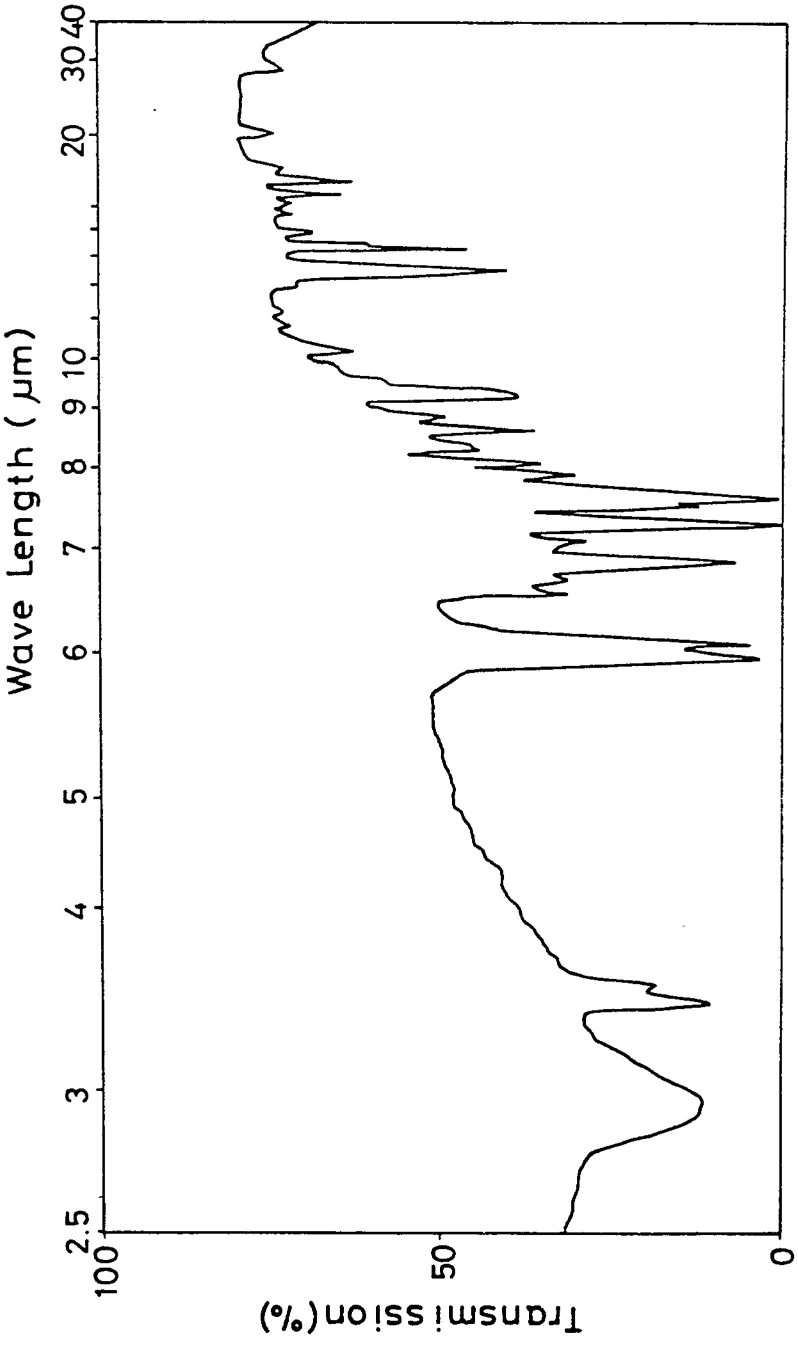

Fig. 6

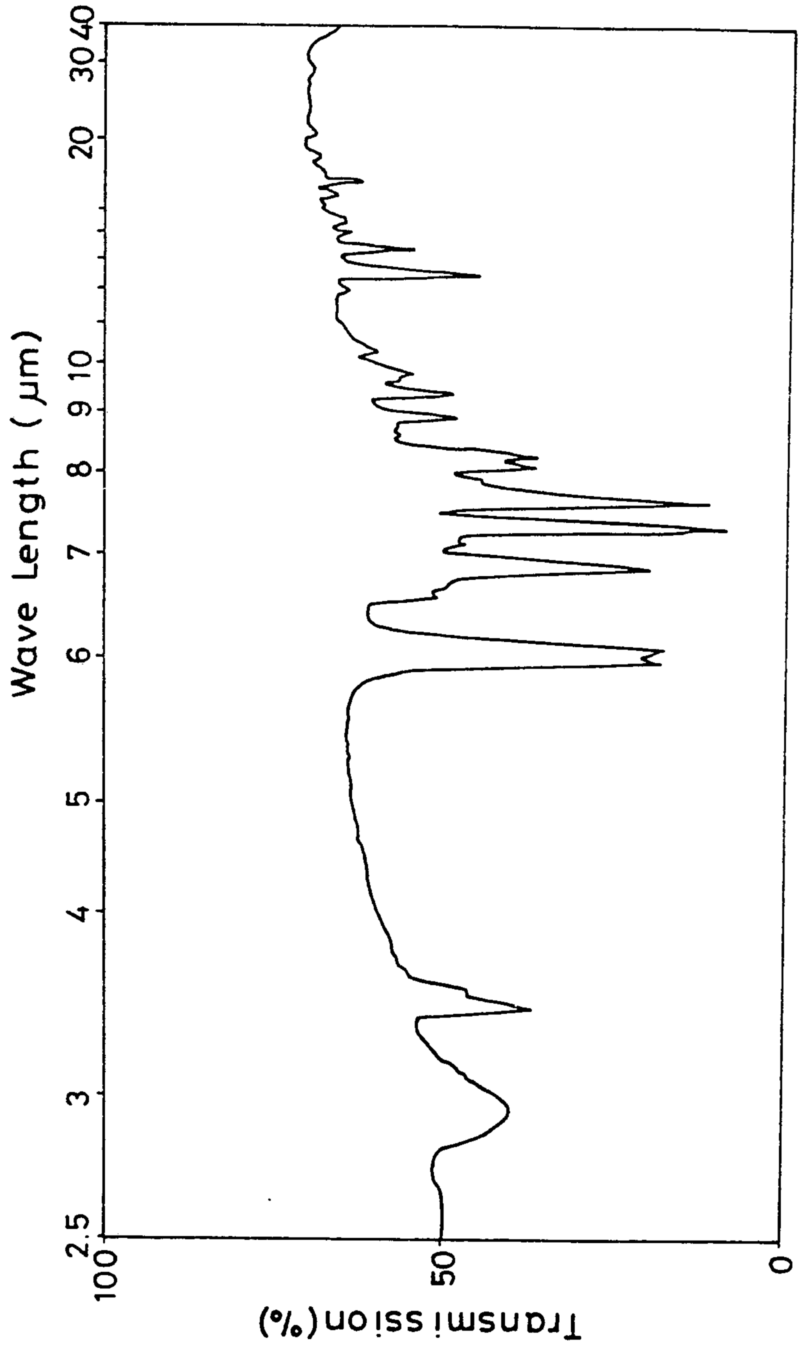

Fig. 7

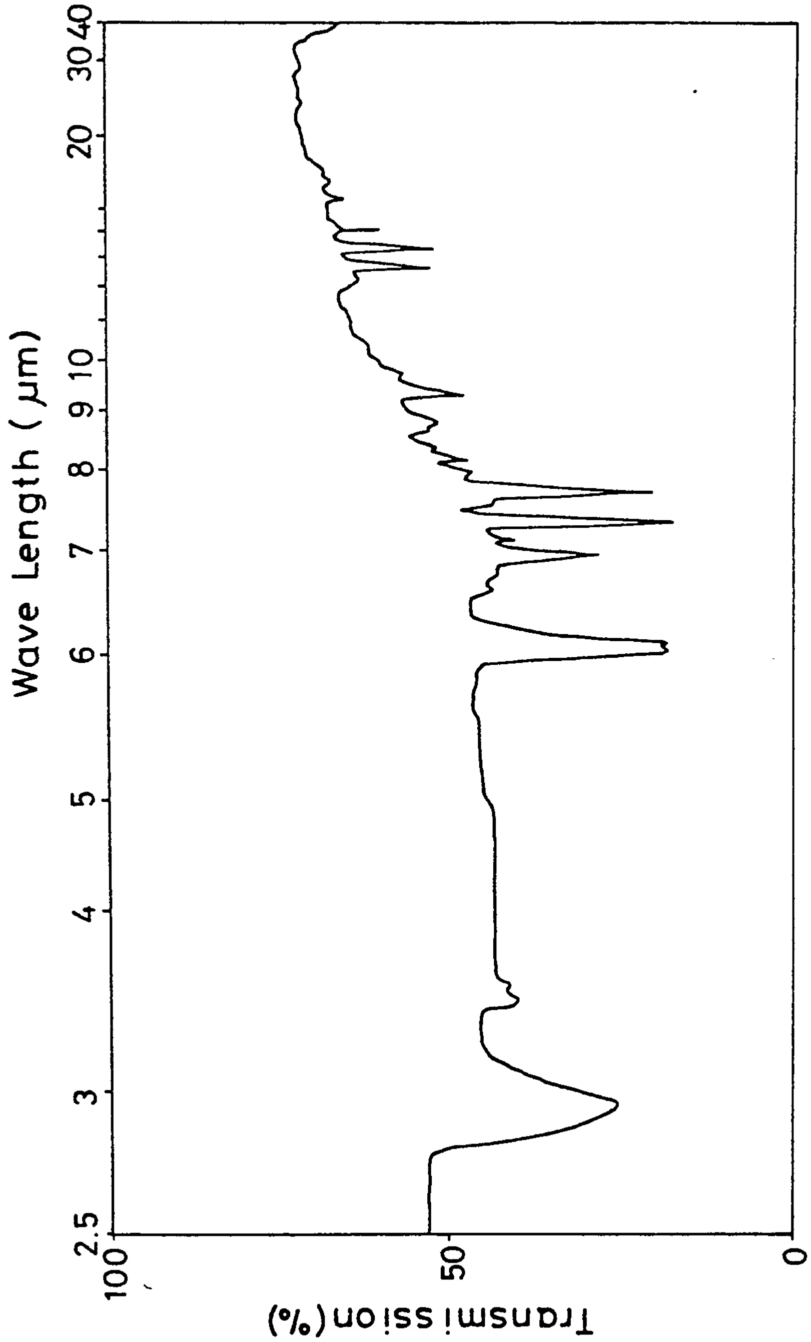

Fig. 8

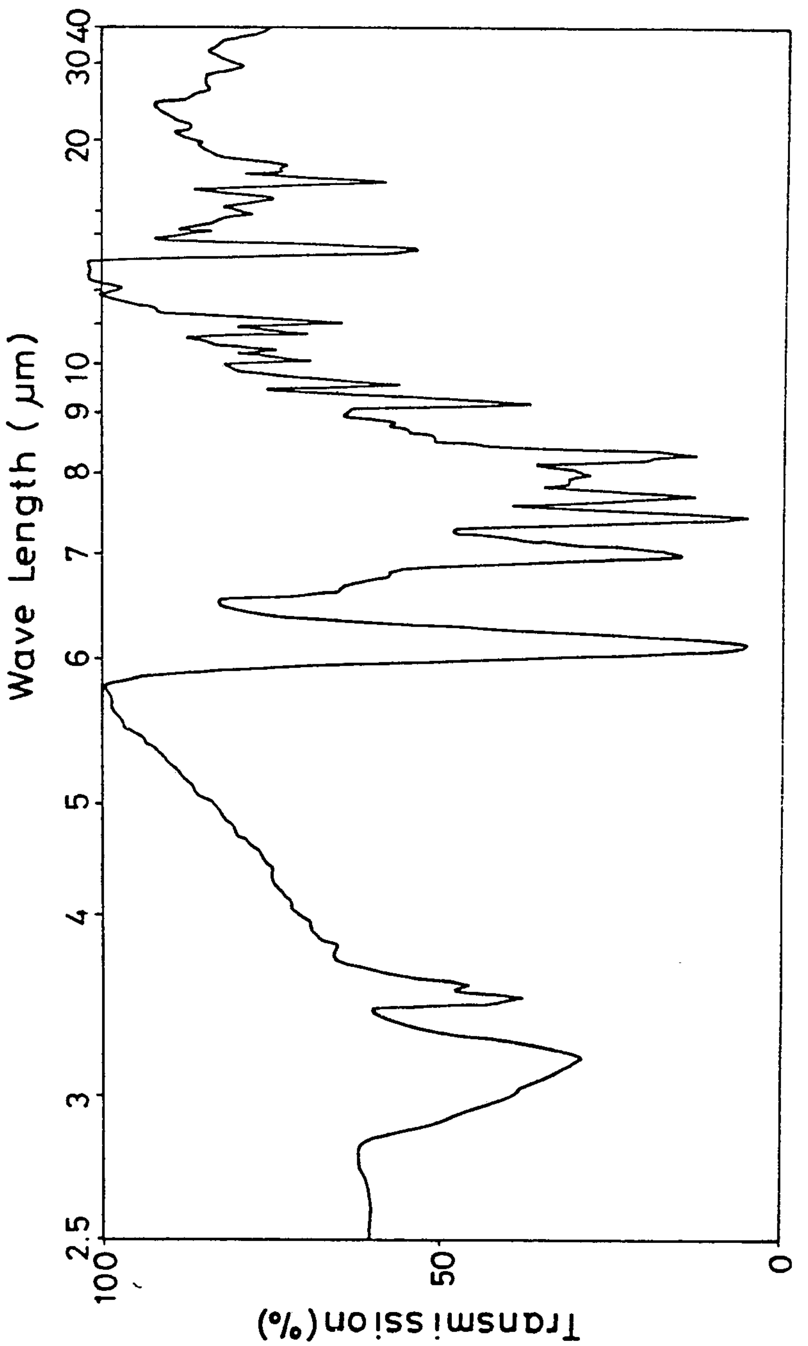

Fig. 9

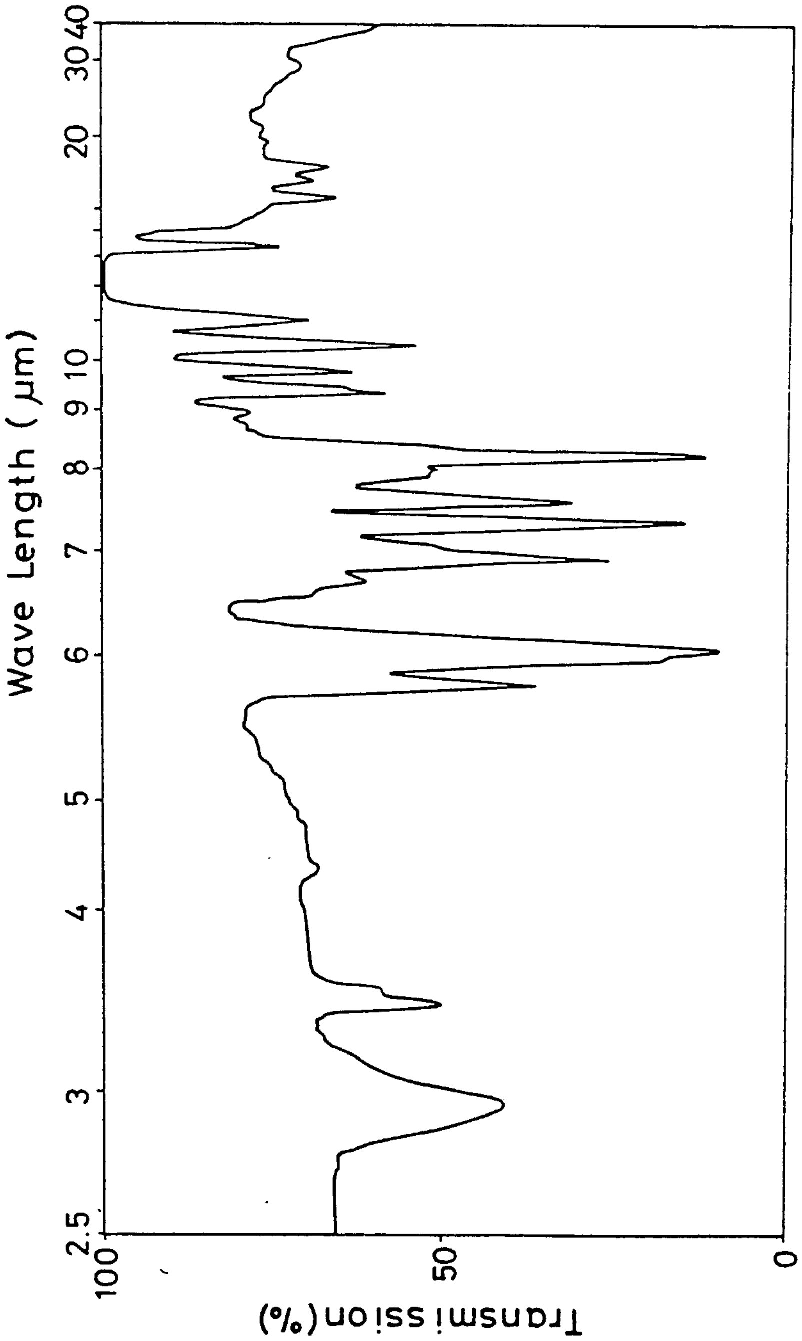

Fig. 10

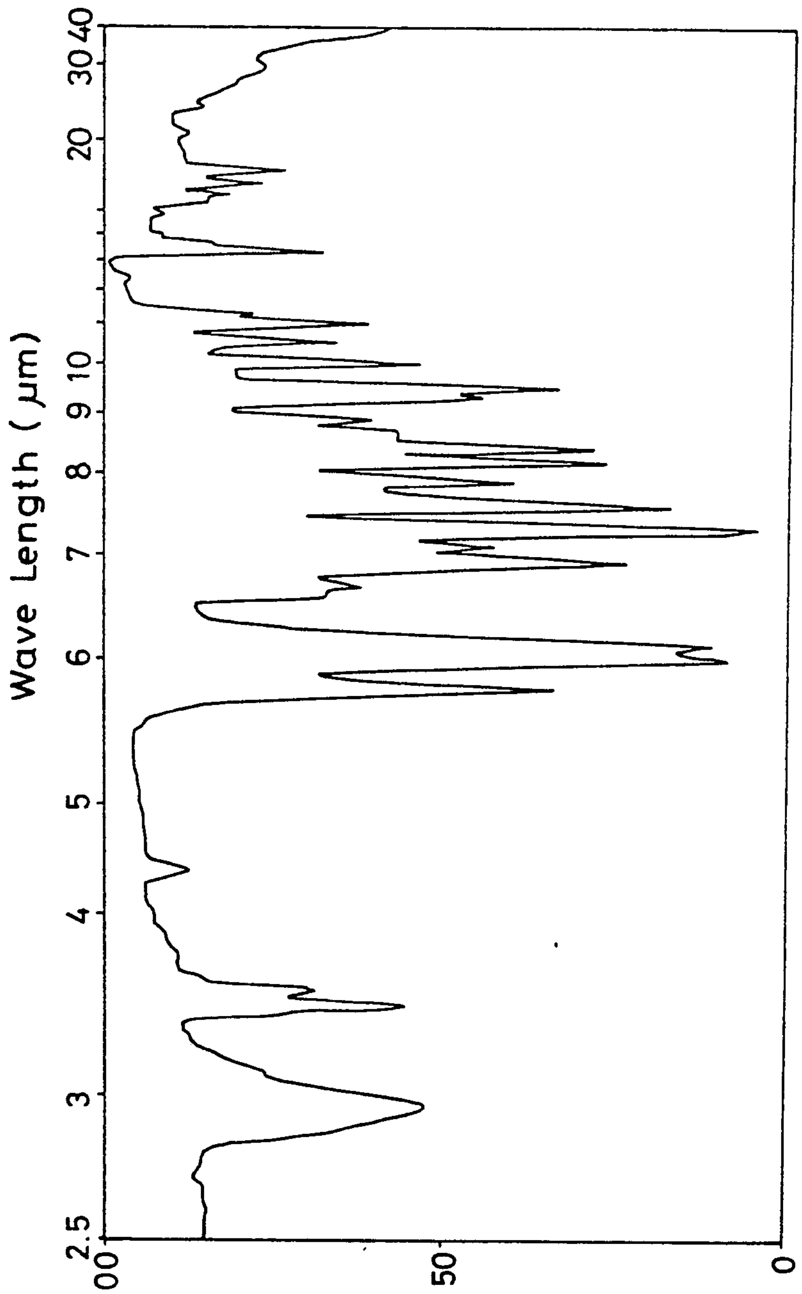

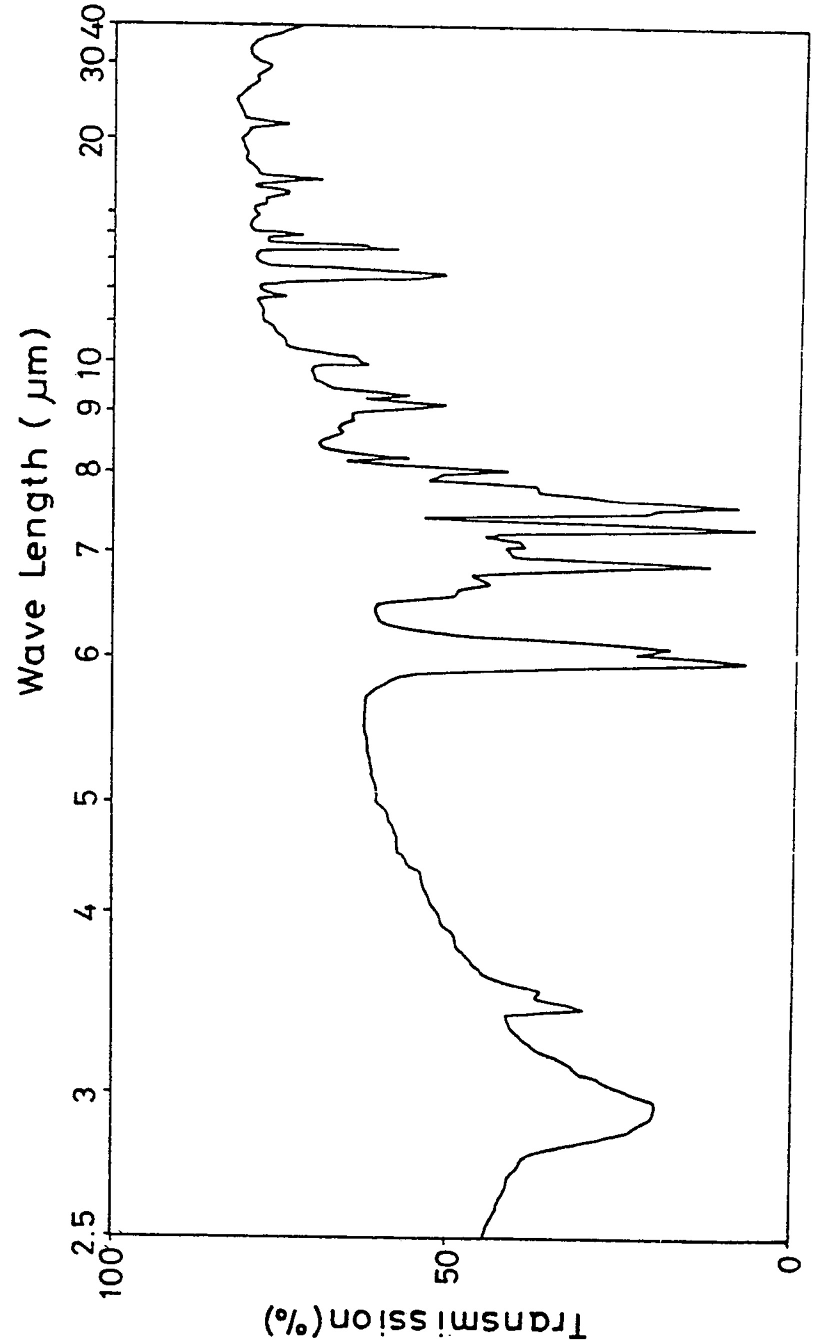


Fig. 11